# FASCICULE DE BREVET EUROPEEN

(11) **EP 1 670 911 B1**
(45) Date de publication et mention de la délivrance du brevet: **04.10.2017**
(21) Numéro de dépôt: 03717485.1
(22) Date de dépôt: 09.04.2003
(51) Int. Cl.: C12N 11/04, C12G 1/022, C12G 1/073, C12C 11/09

(54) **PROCEDE D'IMMOBILISATION DE MICRO-ORGANISMES, PRODUIT CORRESPONDANT ET UTILISATION DE CELUI-CI**
VERFAHREN ZUR IMMOBILISIERUNG VON MIKROORGANISMEN, ENTSPRECHENDES PRODUKT UND DESSEN VERWENDUNG
METHOD FOR IMMOBILISING MICROORGANISMS, RELATED MATERIAL, AND USE THEREOF

(43) Date de publication de la demande: 21.06.2006
(73) Titulaire: Proenol Industria Biotecnologica, Lda, 4405-194 Canelas (PT)
(72) Inventeur: DE FATIMA TEIXEIRA CARDOSO DA SILVA, Maria, P-4405-194 Canelas VNG (PT); STREHAIANO, Pierre, Marie, Jean, F-31750 Escalquens (FR); CHEDAS SAMPAIO, Rui, P-2775 Parede (PT)
(74) Mandataire: Pelayo de Sousa Henriques, Rui
(86) Numéro de dépôt international: PCT/IB2003/001753
(87) Numéro de publication internationale: WO 2004/090128

(56) Documents cités:
- EP-A- 0 350 374
- EP-A- 0 578 617
- WO-A-92/14544
- FR-A- 2 519 022
- FR-A- 2 812 655
- YOKOTSUKA K ET AL: "CONTROLLED SIMULTANEOUS DEACIDIFICATION AND ALCOHOL FERMENTATION OF A HIGH-ACID GRAPE MUST USING TWO IMMOBILIZED YEASTS, SCHIZOSACCHAROMYCES POMBE AND SACCHAROMYCES CEREVISIAE" AMERICAN JOURNAL OF ENOLOGY AND VITICULTURE, XX, XX, vol. 44, no. 4, 1993, pages 371-377, XP000956002 ISSN: 0002-9254
- DIVIES CHARLES ET AL: "Theme 4: Immobilized cell technology in wine production." CRITICAL REVIEWS IN BIOTECHNOLOGY, vol. 14, no. 2, 1994, pages 135-153, XP009017645 ISSN: 0738-8551
- LESTAN DOMEN ET AL: "Development of fungal inocula for bioaugmentation of contaminated soils", APPLIED AND ENVIRONMENTAL MICROBIOLOGY, vol. 62, no. 6, 1996, pages 2045-2052, ISSN: 0099-2240
- WEIR S C ET AL: "Nutrient-enhanced survival of and phenanthrene mineralization by alginate-encapsulated and free Pseudomonas sp. UG14Lr cell in creosote-contaminated soil slurries", APPLIED MICROBIOLOGY AND BIOTECHNOLOGY, SPRINGER VERLAG, BERLIN, DE, vol. 43, no. 5, 1 January 1995 (1995-01-01), pages 946-951, XP008144428, ISSN: 0175-7598
- LOOMIS A K ET AL: "Alginate encapsulation of the white rot fungus Phanerochaete chrysosporium", CURRENT MICROBIOLOGY, vol. 34, no. 2, 1997, pages 127-130, ISSN: 0343-8651
- BASHAN Y: "ALGINATE BEADS AS SYNTHETIC INOCULANT CARRIERS FOR SLOW RELEASE OF BACTERIA THAT AFFECT PLANT GROWTH", APPLIED AND ENVIRONMENTAL MICROBIOLOGY, vol. 51, no. 5, 1986, pages 1089-1098, ISSN: 0099-2240

## Description

### Champ d'application de l'invention

La présente invention propose un procédé nouveau et innovateur pour l'immobilisation de micro-organismes (en particulier, levures ou bactéries) le respectif produit et son utilisation dans des procédés de fermentation ou de bioconversion.

En particulier, cette invention concerne l'Immobilisation de micro-organismes dans des sphères de polymères constituées par deux ou plusieurs couches, ces sphères étant susceptibles d'être déshydratées.

Ces sphères peuvent être utilisées dans l'élaboration de boissons fermentées, ou dans la réalisation de réactions de bioconversion. On appelle réactions de bioconversion aux transformations provoquées par les micro--organismes, mais qui ne nécessitent pas du développement de ces micro--organismes mêmes. Sont particulièrement visées les applications dans le domaine des boissons fermentées comme, notamment: les vins tranquilles, la bière, les vins mousseux, les boissons gazeuses, l'hydromel et l'alcool (éthylique),

### Arrière-plan technologique

Il est bien connu qu'il existe diverses façons d'immobiliser des micro--organismes :
- Des sphères homogènes, ou encore appelées « monocouche » ou « couche simple ». Elles sont constituées par un gel qui contient les micro-organismes, étant décrites dans les documents de brevet : FR2320 349 et FR2359202. Il est connu que ces sphères n'empêchent pas la sortie des micro-organismes (appelé relargage) vers le milieu de réaction, ce qui dans certains cas n'est pas compatible avec les exigences du procédé (vins mousseux par exemple, à cause du trouble indésirable que le relargage occasionne).
- Le brevet EP0173915 décrit la réalisation d'une couche stérile autour du noyau de gel qui contient les micro-organismes, destinée à éviter ce phénomène de relargage, et développe l'application des sphères ou fils obtenus, appelés « double couche », dans les vins mousseux. Ce type de produit peut être humide ou partiellement déshydraté, comme expliqué au brevet EP0350374.

Le problème qui se pose actuellement est que, comme il est connu et prouvé, dans les sphères qui présentent une telle structure (mono-couche ou double-couche) seulement une petite fraction de la population totale de micro-organismes sont réellement en activité. En effet, les substrats nutritifs sont consommés par les micro-organismes situés dans la périphérie de la couche. D'autre part, pour certains cas, le milieu ne contient pas tous les éléments nutritifs nécessaires à la bonne activité de ces micro-organismes immobilisés. Évidemment, ce problème est commun aux biocatalyseurs configurés sous d'autres formes, comme ceux présentés sous la forme de fils comme il est décrit dans le dit document EP0173915. Dans le cas des biocatalyseurs où les micro-organismes se trouvent immobilisés, ceux qui se trouvent à la périphérie ou plus proche de celle-ci (dans le cas de l'existence d'une couche protectrice externe sans micro-organismes) consomment la plupart des éléments nutritifs qui viennent de l'extérieur, rendant impossible ou diminuant l'accès aux micro-organismes situés plus vers l'intérieur ce qui fait que ces micro-organismes restent relativement Inactifs.

### Description de l'invention

Malgré le fait que la description qui suit soit basée sur le cas d'un produit avec les micro-organismes immobilisés, dorénavant nommé biocatalyseur, se trouvant sous forme de sphères, cette forme étant utilisée à titre d'exemple, on peut matérialiser cette invention sous n'importe quelle autre forme, fils ou plaques, dès quelle soit compatible avec les principes généraux énoncés et avec le champ d'application défini dans les revendications.

Selon la présente invention, le problème énoncé précédemment est résolu au moyen d'un produit conformément à la revendication 19 et d'un procédé selon la revendication 1 ainsi que d'utilisations conformément aux revendications 27 et 28.

Selon un mode de réalisation particulier, le problème énoncé précédemment est additionnement résolu au moyen de la création d'une réserve nutritive au centre de la sphère et du placement des micro-organismes dans une couche contiguë à celle-ci, l'ensemble étant suivi par le recouvrement au moyen d'une couche stérile qui définit, dans ce cas, une sphère à trois couches.

De la même façon que ce qui est Indiqué au-dessus pour le biocatalyseur, la description qui suit avec les 2 ou 3 couches est utilisée à titre d'exemple, constituant, en général, la forme préférée d'exécution, une fois qu'il peut se concevoir, selon le principe général de la présente invention, un biocatalyseur multi-couches, avec plus de couches, en alternant par exemple les couches de réserves nutritives, avec les couches qui contiennent les micro-organismes, l'ensemble étant entouré par une couche stérile. Bien que les couches doivent être de préférence concentriques, cela n'est pas obligatoire.

De cette façon, la réserve nutritive peut être configurée comme un groupe d'endroits distincts entourés par la couche qui inclue les micro-organismes, l'ensemble étant entouré par une couche stérile.

Selon une forme préférée d'exécution, l'objet-de cette invention est une sphère à triple couche avec les caractéristiques suivantes
1. Une réserve nutritive qui a pour but de fournir aux micro--organismes immobilisés, dans la couche contiguë, les éléments nutritifs qui leur assurent une bonne activité mais dont le milieu où seront introduites les sphères n'est pas capable de fournir en quantité suffisante. Ces éléments nutritifs peuvent être légèrement différents en nature et en concentration selon la législation en vigueur dans les pays d'utilisation ; mais d'une façon générale ils fournissent une source d'azote (ammoniacal, aminé, selon le micro--organisme), des sels minéraux (phosphate, sulfates, potassium, magnésium, etc.) des oligo-éléments (fer, cuivre, zinc, etc.) et des vitamines (thiamine, biotine, etc.). Les extraits de levures autolysés seront avantageusement utilisés en tant que source nutritive.
   Selon le type d'utilisation, cette réserve nutritive peut aussi contenir un substrat carboné comme un sucre fermentaire ou encore un milieu naturel complet comme un moût de raisin dilué ou pas.
   Selon cette invention, cette solution nutritive est mélangée avec un polymère susceptible de se transformer en gel, par exemple un alginate de sodium. Une concentration adéquate de ce polymère se situe entre 1% et 3% en solution aqueuse.
2. Une couche contiguë à la réserve nutritive constituée d'une solution susceptible de se transformer en gel (dorénavant nommé milieu de fixation) et d'une population de micro-organismes en suspension dans cette solution. Les micro-organismes peuvent être, notamment, des levures ou des bactéries. Pour une application du type fermentation en bouteille de vin mousseux, ou de reprise de fermentation de moûts en arrêt de fermentation, il convient d'utiliser des souches sélectionnées de levures *Saccharomyces cerevisiae, Saccharomyces uvarum.*
   Mis à part *Saccharomyces,* on peut aussi utiliser d'autres genres de levures, par exemple dans l'élaboration de bière, ou de boissons de faible teneur alcoolique ou encore dans les réactions de bioconversion. Par exemple, le genre *Schizosaccharomyces* sera plus utilisé pour la désacidification de moût de raisin acide, les bactéries *Oenococcus oeni* ou *Lactobacillus* pour la fermentation malolactique et le genre Candida pour la bioconversion de xylose en xylitol.
   Selon une autre caractéristique de l'invention, la concentration du polymère varie entre 1 % et 50%.
   Les micro-organismes peuvent venir d'une culture fraîche réalisée selon les normes en vigueur à l'industrie ou bien d'une préparation commerciale sèche active ou lyophilisée et utilisée selon les conseils du fabricant.
3. Une couche externe additionnelle qui est élaborée à partir d'un polymère de nature identique aux deux autres couches et susceptible de se transformer en gel. La concentration du polymère est identique à celle utilisée pour les autres couches. Selon une caractéristique supplémentaire de l'invention, dans la couche externe, dans certaines applications, une préparation enzymatique ou une préparation de composants organiques pourra être introduite :
   Par exemple (i), une solution de lysozyme additionnée dans la couche externe pour empêcher le développement de bactéries indésirables qui sont sensibles à cette enzyme (exemple connu des bactéries lactiques en vinification) et qui pourraient être à l'origine d'un trouble difficile à éliminer dans le cas des vins mousseux.
   Par exemple (li), les parois de levures qui vont fixer les acides gras, inhibiteurs de l'activité des micro--organismes fixés, dans le cas des traitements d'arrêt de fermentation.

Selon une autre caractéristique de l'invention les particules ainsi constituées, appelées de sphères triple couche, sont produites dans une seule étape.

La réalisation simultanée de trois couches concentriques peut être faite recourrant à un dispositif de tubes concentriques qui définit deux zones annulaires concentriques autour d'une zone centrale également concentrique, faisant l'incorporation de la réserve nutritive par le tube central, l'incorporation des micro--organismes et du milieu de fixation, à travers de la zone annulaire définie par la partie externe du tube central et par la partie interne du tube intermédiaire et en faisant l'incorporation de la couche externe à travers de la zone annulaire définie par la partie externe du tube intermédiaire et par la partie interne du tube externe.

Le diamètre des sphères humides est compris entre 1 et 5 mm. La gélification est faite au moyen du passage d'un agent de réticulation, notamment le chlorure de calcium, sur une solution selon une procédure déjà connue, et préférentielle, où la réticulation de la sphère est faite de l'extérieur vers l'intérieur. Selon une autre caractéristique de l'invention, ces sphères peuvent être plus ou moins déshydratées jusqu'à une activité de l'eau (AW) finale de 0,1 à 0,5, de préférence de 0,3 à 0,4, Cette déshydratation est réalisée avec une technique de séchage, à lit fluidisé ou l'utilisation d'étuve. Ces sphères sont conservées dans un emballage résistant à la vapeur et à l'air et de préférence maintenu à une température relativement basse, étant l'idéale 4° C, Dans ces conditions il est possible de conserver le produit plusieurs mois.

Les exemples qui suivent illustrent quelques-unes des applications, caractéristiques et avantages de l'invention.

### Exemple 1 : Préparation de sphères avec triple couche d'alginate, contenant des levures Saccharomyces cerevisiae, immobilisées et au préalable préparées pour résister à un milieu riche en alcool

Les sphères sont préparées à partir d'une solution d'alginate de sodium, polymère linéaire extrait d'algues et composé par l'acide α-D-manuronique et β-D-guluronique. Les levures, au préalable acclimatées à l'alcool ou non, sont mélangées avec une partie de cette solution dans une cuve. Pour cet exemple il sera utilisé une souche de Saccharomyces cerevisiae EC1118 commercialisée sous la marque Lalvin.

Ensuite et grâce à un dispositif de tubes concentriques, cette solution passe par un système de vibration qui permet la formation de sphères.

Elles sont tout de suite gélifiées lors de la mise en contact avec une solution 0,2 M de chlorure de calcium, étant le temps de contact d'une demi--heure.

Les sphères ainsi formées sont lavées par immersion pendant 10 minutes dans de l'eau déminéralisée.

Ensuite, les sphères sont déshydratées partiellement en lit fluidisé. Ce séchage permet d'obtenir une activité de l'eau (AW) comprise entre 0,3 et 0,4. La température de séchage est inférieure ou égale à 40°C. Le diamètre des sphères qui sont obtenues est de 1,5 à 4 mm. Après le contrôle qualité, les levures immobilisées et déshydratées sont emballées et stockées à 4°C avant leur utilisation.

### Exemple 2: Stabilité des levures pendant le stockage

Afin de valider la préservation de l'activité des sphères, possédant la couche de *Saccharomyces cerevisiae,* suite à une conservation de 6 mois à 4 °C, le contrôle est réalisé de la façon suivante :
- 30 g de sphères préparées selon l'exemple 1, recouvertes d'une solution de saccharose à 50gl-1 (volume 100ml) à 37°C.
- La solution est maintenue à 37°C et l'évolution de la concentration en sucre est à suivre au long du temps.
- Après deux heures la concentration du sucre est inférieure à 2gl⁻¹, ce qui correspond à la fin de fermentation.

Ce contrôle de référence est effectué au temps 0 (immédiatement après la production des sphères) et aussi après 6 mois de stockage à 4°C. La fin de la fermentation est effective après le même temps de fermentation de 2 heures. Donc les levures immobilisées préparées selon l'exemple 1, paraissent être stables pendant 6 mois de stockage à 4°C.

## Revendications

1. Procédé d'immobilisation de micro-organismes **caractérisé en ce qu'**on incorpore dans un milieu de fixation des micro-organismes une réserve nutritive physiquement concentrée dans une zone ou physiquement séparée dans des zones distinctes, ladite réserve étant adéquate aux dits micro-organismes et se positionnant dans ledit milieu de fixation des micro-organismes avec une densité plus grande dans les zones plus distantes de la frontière externe du dit milieu, **en ce qu'**on réalise une réticulation de ce milieu de support **et en ce que** on additionne une couche externe stérile sans micro-organismes et imperméable aux micro-organismes cités.

2. Procédé selon la revendication 1, **caractérisé en ce que** la réserve nutritive inclue les éléments nutritifs fournissant, notamment, une source d'azote (ammoniacale ou aminé selon les micro-organismes), sels minéraux (entre autres, phosphates, sulfates, potassium, magnésium) oligo-éléments (entre autres, fer, cuivre, zinc) et vitamines (entre autres, thiamine, biotine).

3. Procédé selon quelconque des revendications antérieures, **caractérisé en ce qu'**on utilise des sources nutritives complexes comme les extraits de levures autolysées.

4. Procédé selon quelconque des revendications antérieures, **caractérisé en ce que** la réserve nutritive contienne un substrat carboné comme un sucre fermentable ou encore un milieu de culture complet comme un moût de raisin dilué ou pas.

5. Procédé selon quelconque des revendications antérieures, **caractérisé en ce que** la réserve nutritive est mélangée à un polymère susceptible de ce transformer en gel, notamment l'alginate de sodium.

6. Procédé selon la revendication antérieure, **caractérisé en ce que** la solution susceptible de se transformer en gel possède une concentration en polymère variant entre 1% et 3%.

7. Procédé selon quelconque des revendications antérieures, **caractérisé en ce que** la ou les couche(s) contiguë(s) à la réserve nutritive est(sont) constituée(s) d'une solution susceptible de se transformer en gel et des micro-organismes en suspension dans cette solution.

8. Procédé selon la revendication antérieure, **caractérisé en ce que** la solution susceptible de se transformer en gel a une concentration en polymère variant entre 1% et 50%.

9. Procédé selon les revendications 7 et 8, **caractérisé en ce que** les micro-organismes sont des levures ou des bactéries.

10. Procédé selon la revendication 9, **caractérisé en ce qu'**on utilise des souches de levures sélectionnées de *Saccharomyces cerevisiae* ou *Saccharomyces uvarum,* lors des fermentations en bouteilles de vins mousseux ou lors de la reprise de fermentation de moûts présentant un ralentissement ou un arrêt de la fermentation alcoolique,

11. Procédé selon la revendication 9, **caractérisé en ce qu'**on utilise des levures du genre *Schlzosaccharomyces,* lors de la désacidification des moûts de raisins acides.

12. Procédé selon la revendication 9, **caractérisé en ce qu'**on utilise des bactéries *Oenococcus oeni* ou *Lactobacillus,* lors de la fermentation malolactique.

13. Procédé selon la revendication 9, **caractérisé en ce qu'**on utilise des levures du genre Candida, lors de la bioconversion du xylose en xylitol.

14. Procédé selon quelconque des revendications antérieures, **caractérisé en ce qu'**on additionne une couche externe stérile, sans micro-organismes et sans perméabilité pour les micro-organismes existant dans le milieu de fixation, au milieu de fixation des micro-organismes, simultanément ou après l'étape de réticulation de celui-ci.

15. Procédé selon la revendication antérieure, **caractérisé en ce que** la couche externe est élaborée à partir d'un polymère d'une nature identique aux couches intérieures et susceptible de se transformer en gel et possédant une concentration identique à celle des autres couches.

16. Procédé selon les revendications 14 et 15, **caractérisé en ce qu'**on introduit une préparation enzymatique ou une préparation de composés organiques, dans la couche externe.

17. Procédé selon la revendication antérieure, **caractérisée en ce qu'**on additionne une solution de lysozyme à la dite couche externe, empêchant le développement d'espèces indésirables qui sont sensibles à cette enzyme, notamment des bactéries lactiques en vinification.

18. Procédé selon la revendication 16, **caractérisé en ce qu'**on additionne à la dite couche externe des parois de levures qui vont fixer les acides gras inhibiteurs de l'activité des micro-organismes fixés, lors du traitement d'arrêt de fermentation.

19. Produit avec des micro-organismes Immobilisés, **caractérisé en ce qu'**il possède une réserve nutritive physiquement concentrée dans une zone ou physiquement séparée dans des zones distinctes, incorporée dans le milieu de fixation des micro-organismes, ladite réserve étant adéquate aux dits micro-organismes et se positionnant dans le dit milieu de fixation des micro-organismes avec une densité plus grande dans les zones plus distantes de la frontière externe du dit milieu, **en ce que** le dit milieu de support est réticulé et **en ce que** le produit possède une couche externe stérile sans micro-organismes et imperméable aux micro-organismes cités.

20. Procédé selon les revendications 1 à 8, pour la production du produit selon la revendication antérieure, **caractérisé en ce que** le produit est fait dans une seule étape avec trois couches, en utilisant des tubes concentriques qui définissent deux zones annulaires concentriques autour d'une zone centrale également concentrique et en réalisant l'incorporation de la réserve nutritive à travers de l'intérieur du tube central, incorporation des micro-organismes et du respectif milieu de fixation, à travers de la zone annulaire définie par la partie externe du tube central et par la partie interne du tube intermédiaire, et en faisant l'incorporation de la couche externe à travers de la zone annulaire définie par la partie externe du tube Intermédiaire et par la partie interne du tube externe.

21. Procédé selon la revendication antérieure, **caractérisé en ce que** la réticulation du produit dispensé par le système de tubes est faite par le passage de celui-ci sur une solution d'un agent de réticulation,

22. Procédé selon la revendication antérieure, **caractérisé en ce que** le polymère susceptible de se transformer en gel est de nature identique dans les trois couches du produit **et en ce que** lorsque celui-ci est un alginate de sodium, l'agent de réticulation est le chlorure de calcium, en se faisant ainsi la réticulation du dit produit exclusivement de l'extérieur vers l'intérieur.

23. Procédé selon quelconque des revendications 20 à 22, **caractérisé en ce que** le produit dispensé par le dispositif de tubes concentriques est coupé par un dispositif de vibration, formant ainsi des sphères.

24. Procédé selon quelconque des revendications 20 à 23, **caractérisé en ce qu'**ensuite le produit subit une déshydratation partielle jusqu'à une AW finale de 0,1 à 0,5, de préférence de 0,3 à 0,4, notamment en utilisant une technique de séchage, à lit fluidisé ou l'utilisation d'étuves.

25. Produit selon la revendication 19, produit selon le procédé de la revendication 23, **caractérisé en ce qu'**il possède une forme sphérique et trois couches.

26. Produit selon la revendication antérieure, **caractérisée en ce que** le diamètre externe des sphères humides est compris entre 1 mm et 5 mm.

27. Utilisation **caractérisée par** l'emploi du produit de quelconque des revendications 19 ou 25 à 26, pour la fermentation de boissons en bouteille.

28. Utilisation **caractérisée par** l'emploi du produit de quelconque des revendications 19 ou 25 à 26, pour la reprise de fermentation de moûts présentant un ralentissement ou un arrêt de la fermentation alcoolique.

## Patentansprüche

1. Verfahren zur Immobilisierung von Mikroorganismen, **dadurch gekennzeichnet, dass** in ein Medium zum Fixieren der Mikroorganismen ein Nährstoffvorrat eingebracht wird, der physisch in einem Bereich konzentriert ist oder physisch in unterschiedliche Bereiche getrennt ist, wobei der Vorrat für die Mikroorganismen geeignet ist und in dem Medium zum Fixieren der Mikroorganismen in einer Dichte angeordnet ist, die in Bereichen größer ist, die von der Außengrenze des Mediums weiter entfernt sind, dass eine Vernetzung des Trägermediums durchgeführt wird und dass eine sterile Außenschicht ohne Mikroorganismen zugegeben wird, die für die Mikroorganismen undurchlässig ist.

2. Verfahren nach Anspruch 1, **dadurch gekennzeichnet, dass** der Nährstoffvorrat Nährstoffe enthält, die insbesondere eine Stickstoffquelle (Ammoniak- oder Aminostickstoff je nach Mikroorganismen), Mineralsalze (unter anderem Phosphate, Sulfate, Kalium, Magnesium), Spurenelemente (unter anderem Eisen, Kupfer, Zink) und Vitamine (unter anderem Thiamin, Biotin) bereitstellen.

3. Verfahren nach einem der vorhergehenden Ansprüche, **dadurch gekennzeichnet, dass** komplexe Nährstoffquellen wie etwa autolysierte Hefeextrakte verwendet werden.

4. Verfahren nach einem der vorhergehenden Ansprüche, **dadurch gekennzeichnet, dass** der Nährstoffvorrat ein kohlenstoffhaltiges Substrat wie etwa einen vergärbaren Zucker oder auch ein komplettes Kulturmedium wie etwa verdünnten oder unverdünnten Traubenmost enthält.

5. Verfahren nach einem der vorhergehenden Ansprüche, **dadurch gekennzeichnet, dass** der Nährstoffvorrat mit einem Polymer, das in ein Gel umwandelbar ist, insbesondere Natriumalginat, gemischt ist.

6. Verfahren nach dem vorhergehenden Anspruch, **dadurch gekennzeichnet, dass** die Lösung, die in ein Gel umwandelbar ist, eine Polymerkonzentration aufweist, die zwischen 1% und 3 % variiert.

7. Verfahren nach einem der vorhergehenden Ansprüche, **dadurch gekennzeichnet, dass** die an den Nährstoffvorrat angrenzende(n) Schicht(en) aus einer in ein Gel umwandelbaren Lösung und in dieser Lösung suspendierten Mikroorganismen besteht/-en.

8. Verfahren nach dem vorhergehenden Anspruch, **dadurch gekennzeichnet, dass** die in ein Gel umwandelbare Lösung eine Polymerkonzentration zwischen 1% und 50% aufweist.

9. Verfahren nach den Ansprüchen 7 und 8, **dadurch gekennzeichnet, dass** es sich bei den Mikroorganismen um Hefen oder Bakterien handelt.

10. Verfahren nach Anspruch 9, **dadurch gekennzeichnet, dass** die ausgewählten Hefestämme *Saccharomyces cerevisiae* oder *Saccharomyces uvarum* zur Flaschengärung von Schaumwein oder zum Neubeimpfen einer Mostgärung, bei der die alkoholische Gärung stockt oder stillsteht, verwendet werden.

11. Verfahren nach Anspruch 9, **dadurch gekennzeichnet, dass** Hefen der Gattung *Schizosaccharomyces* zur Entsäuerung von sauren Traubenmosten verwendet werden.

12. Verfahren nach Anspruch 9, **dadurch gekennzeichnet, dass** die Bakterien *Oenococcus oeni* oder *Lactobacillus* zur malolaktischen Gärung verwendet werden.

13. Verfahren nach Anspruch 9, **dadurch gekennzeichnet, dass** Hefen der Gattung Candida zur Biokonversion von Xylose in Xylit verwendet werden.

14. Verfahren nach einem der vorhergehenden Ansprüche, **dadurch gekennzeichnet, dass** eine sterile Außenschicht ohne Mikroorganismen und ohne Durchlässigkeit für im Fixierungsmedium vorhandene Mikroorganismen dem Medium zum Fixieren der Mikroorganismen gleichzeitig mit oder nach dem Vernetzungsschritt derselben zugegeben wird.

15. Verfahren nach dem vorhergehenden Anspruch, **dadurch gekennzeichnet, dass** die Außenschicht aus einem Polymer hergestellt ist, das von identischer Natur wie die Innenschichten und in ein Gel umwandelbar ist und eine Konzentration aufweist, die zu jener der anderen Schichten identisch ist.

16. Verfahren nach den Ansprüchen 14 und 15, **dadurch gekennzeichnet, dass** ein Enzympräparat oder ein Präparat aus organischen Verbindungen in die Außenschicht eingebracht wird.

17. Verfahren nach dem vorhergehenden Anspruch, **dadurch gekennzeichnet, dass** eine Lysozymlösung zu der Außenschicht hinzugegeben wird, welche die Entwicklung von unerwünschten Spezies, die auf dieses Enzym empfindlich reagieren, insbesondere Milchsäurebakterien bei der Weinherstellung, verhindert.

18. Verfahren nach Anspruch 16, **dadurch gekennzeichnet, dass** der Außenschicht Hefezellwände zugegeben werden, die Fettsäuren, welche die Aktivität der fixierten Mikroorganismen hemmen, bei Behandlung eines Gärstopps fixieren werden.

19. Produkt mit immobilisierten Mikroorganismen, **dadurch gekennzeichnet, dass** es einen in das Medium zum Fixieren der Mikroorganismen eingebrachten Nährstoffvorrat aufweist, der physisch in einem Bereich konzentriert ist oder physisch in unterschiedliche Bereiche getrennt ist, wobei der Vorrat für die Mikroorganismen geeignet ist und in dem Medium zum Fixieren der Mikroorganismen in einer Dichte angeordnet ist, die in Bereichen größer ist, die von der Außengrenze des Mediums weiter entfernt sind, dass das Trägermedium vernetzt ist und dass das Produkt eine sterile Außenschicht ohne Mikroorganismen aufweist, die für die Mikroorganismen undurchlässig ist.

20. Verfahren nach den Ansprüchen 1 bis 18 zur Herstellung des Produktes nach dem vorhergehenden Anspruch, **dadurch gekennzeichnet, dass** das Produkt in einem einzigen Schritt mit drei Schichten ausgebildet wird durch Verwenden von konzentrischen Rohren, die zwei konzentrische ringförmige Bereiche um einen ebenfalls konzentrischen zentralen Bereich definieren, und durch Durchführen des Einbringens des Nährstoffvorrats über das Innere des zentralen Rohrs, des Einbringens der Mikroorganismen und des entsprechenden Fixierungsmediums über den ringförmigen Bereich, der durch den äußeren Abschnitt des zentralen Rohrs und durch den inneren Abschnitt des Zwischenrohrs definiert ist, und durch Durchführen des Einbringens der Außenschicht über den ringförmigen Bereich, der durch den äußeren Abschnitt des Zwischenrohrs und durch den inneren Abschnitt des Außenrohrs definiert ist.

21. Verfahren nach dem vorhergehenden Anspruch, **dadurch gekennzeichnet, dass** die Vernetzung des durch das Rohrsystem ausgegebenen Produktes durch Leiten desselben über ein Vernetzungsmittel erfolgt.

22. Verfahren nach dem vorhergehenden Anspruch, **dadurch gekennzeichnet, dass** das in ein Gel umwandelbare Polymer von identischer Natur in den drei Schichten des Produktes ist und dass, wenn es sich dabei um ein Natriumalginat handelt, das Vernetzungsmittel Calciumchlorid ist, wodurch die Vernetzung des Produktes ausschließlich von außen nach innen erfolgt.

23. Verfahren nach einem der Ansprüche 20 bis 22, **dadurch gekennzeichnet, dass** das durch die Vorrichtung mit konzentrischen Rohren ausgegebene Produkt durch eine Vibrationsvorrichtung abgeschnitten wird, sodass Kügelchen gebildet werden.

24. Verfahren nach einem der Ansprüche 20 bis 23, **dadurch gekennzeichnet, dass** das Produkt anschließend eine teilweise Dehydratation auf einen Aw-Endwert von 0,1 bis 0,5, bevorzugt 0,3 bis 0,4, insbesondere unter Verwendung einer Trockentechnik im Fließbett oder unter Verwendung von Öfen durchläuft.

25. Produkt nach Anspruch 19, Produkt gemäß dem Verfahren nach Anspruch 23, **dadurch gekennzeichnet, dass** es eine Kugelform und drei Schichten aufweist,

26. Produkt nach dem vorhergehenden Anspruch, **dadurch gekennzeichnet, dass** der Außendurchmesser der feuchten Kügelchen zwischen 1 mm und 5 mm liegt.

27. Verwendung, **gekennzeichnet durch** den Einsatz des Produktes nach einem der Ansprüche 19 oder 25 bis 26 zur Flaschengärung von Getränken.

28. Verwendung, **gekennzeichnet durch** den Einsatz des Produktes nach einem der Ansprüche 19 oder 25 bis 26 zum Neubeimpfen einer Mostgärung, bei der die alkoholische Gärung stockt oder stillsteht.

## Claims

1. A method for immobilizing microorganisms, **characterized in that** one incorporates, in a medium for fixing the microorganisms, a nutrient reserve physically concentrated in one zone or physically separated in different zones, said reserve being suitable for said microorganisms and being positioned in said medium for fixing the microorganisms with a greater density in the zones that are at a greater distance from the external boundary of said medium, **in that** a crosslinking of this support medium is carried out, and **in that** one adds a sterile external layer without microorganisms and impermeable to said microorganisms.

2. The method according to Claim 1, **characterized in that** the nutrient reserve includes the nutrient elements supplying, in particular, a nitrogen source (ammoniacal or aminated depending on the microorganisms), mineral salts (including phosphates, sulfates, potassium, magnesium), trace elements (including iron, copper, zinc) and vitamins (including thiamine, biotin).

3. The method according to either of the preceding claims, **characterized in that** one uses complex nutrient sources as the extract of autolysed yeasts.

4. The method according to any one of the preceding claims, **characterized in that** the nutrient reserve contains a carbon substrate as a fermentable sugar or a complete culture medium such as a diluted or undiluted grape must.

5. The method according to any one of the preceding claims, **characterized in that** the nutrient reserve is mixed with a polymer capable of being transformed into a gel, in particular sodium alginate.

6. The method according to the preceding claim, **characterized in that** the solution capable of being transformed into a gel has a polymer concentration varying between 1% and 3%.

7. The method according to any one of the preceding claims, **characterized in that** the layer(s) contiguous to the nutrient reserve consist(s) of a solution capable of being transformed into a gel and or the microorganisms suspended in this solution.

8. The method according to the preceding claim, **characterized in that** the solution capable of being transformed into a gel has a polymer concentration varying between 1% and 50%.

9. The method according to Claims 7 and 8, **characterized in that** the microorganisms are yeasts or bacteria.

10. The method according to Claim 9, **characterized in that** one uses yeast strains selected from *Saccharomyces cerevisiae* or *Saccharomyces uvarum,* in fermentations in bottles of sparkling wines or in the resumption of fermentation of musts exhibiting a slowing or stopping of alcoholic fermentation.

11. The method according to Claim 9, **characterized in that** yeasts of the genus *Schizosaccharomyces* are used in the deacidification of the acidic grape musts.

12. The method according to Claim 9, **characterized in that** *Oenococcus* oeni or *Lactobacillus* bacteria are used in the malolactic fermentation.

13. The method according to Claim 9, **characterized in that** yeasts of the genus Candida are used in the bioconversion of xylose to xylitol.

14. The method according to any one of the preceding claims, **characterized in that** a sterile external layer without microorganisms and without permeability for the microorganisms existing in the fixation medium is added to the medium for fixing microorganisms, simultaneously or after the step of crosslinking said medium.

15. The method according to the preceding claim, **characterized in that** the external layer is produced from a polymer of identical nature to that of the inner layers and which is capable of being transformed into a gel and having a concentration identical to that of the other layers.

16. The method according to Claims 14 and 15, **characterized in that** one introduces an enzymatic preparation or a preparation of organic compounds into the external layer.

17. The method according to the preceding claim, **characterized in that** a lysozyme solution is added to said external layer, preventing the development of undesirable species that are sensitive to this enzyme, in particular, lactic bacteria in winemaking.

18. The method according to Claim 16, **characterized in that**, to said external layer, one adds yeast walls that will fix the fatty acids inhibiting the activity of the fixed microorganisms, during the fermentation stopping treatment.

19. A product with immobilized microorganisms, **characterized in that** it has a nutrient reserve physically concentrated in one zone or physically separated in different zones, incorporated in the medium for fixing the microorganisms, said reserve being suitable for said microorganisms and being positioned in said medium for fixing the microorganisms with a greater density in the zones that are at a greater distance from the external boundary of said medium, **in that** said support medium is crosslinked, and **in that** the product has a sterile external layer without microorganisms and impermeable to said microorganisms.

20. The method according to Claims 1 to 18, for the production of the product according to the preceding claim, **characterized in that** the product is made in a single step with three layers, using concentric tubes that define two concentric annular zones around a central zone which is also concentric, and by carrying out the incorporation of the nutrient reserve through the interior of the central tube, the incorporation of the microorganisms and of the respective fixing medium, through the annular zone defined by the external portion of the central tube and by the internal portion of the intermediate tube, and by carrying out the incorporation of the external layer through the annular zone defined by the external portion of the intermediate tube and by the internal portion of the external tube.

21. The method according to the preceding claim, **characterized in that** the crosslinking of the product dispensed by the system of tubes is done by passing said product through a solution of a crosslinking agent.

22. The method according to the preceding claim, **characterized in that** the polymer capable of being transformed into a gel is of identical nature in the three layers of the product, and **in that**, when the polymer is a sodium alginate, the crosslinking agent is calcium chloride, the crosslinking of said product thus taking place exclusively from outside to inside.

23. The method according to any one of Claims 20 to 22, **characterized in that** the product dispensed by the device consisting of concentric tubes is cut by a vibration device, thus forming spheres.

24. The method according to any one of Claims 20 to 23, **characterized in that** the product then undergoes a partial dehydration to a final a_{w} of 0.1 to 0.5, preferably of 0.3 to 0.4, in particular by using a drying technique, with fluidized bed or the use of autoclaves.

25. The product according to Claim 19, produced according to the method of Claim 23, **characterized in that** it has a spherical shape and three layers.

26. The product according to the preceding claim, **characterized in that** the external diameter of the moist spheres is between 1 mm and 5 mm.

27. A use **characterized by** the use of the product of any one of Claims 19 or 25 to 26, for the fermentation of drinks in a bottle.

28. The use **characterized by** the use of the product of any one of Claims 19 or 25 to 26, for the resumption of the fermentation of musts exhibiting a slowing or stopping of the alcoholic fermentation.
